# EUROPÄISCHE PATENTSCHRIFT

(11) **EP 1 664 004 B1**
(45) Veröffentlichungstag und Bekanntmachung des Hinweises auf die Patenterteilung: **24.06.2015**
(21) Anmeldenummer: 04764990.0
(22) Anmeldetag: 09.09.2004
(51) Int. Cl.: C07D 265/32, C07D 409/14

(54) **VERFAHREN ZUR HERSTELLUNG VON 4-(4-AMINOPHENYL)-3-MORPHOLINON**
METHOD FOR THE PRODUCTION OF 4-(4-AMINOPHENYL)-3-MORPHOLINON
PROCEDE DE PRODUCTION DE 4-(4-AMINOPHENYL)-3-MORPHOLINONE

(30) Priorität: 15.09.2003 DE 10342570
(43) Veröffentlichungstag der Anmeldung: 07.06.2006
(73) Patentinhaber: Bayer Intellectual Property GmbH, 40789 Monheim (DE)
(72) Erfinder: THOMAS, Christian, 42113 Wuppertal (DE); BERWE, Mathias, 45549 Sprockhövel (DE); STRAUB, Alexander, 42117 Wuppertal (DE)
(74) Vertreter: BIP Patents
(86) Internationale Anmeldenummer: PCT/EP2004/010054
(87) Internationale Veröffentlichungsnummer: WO 2005/026135

(56) Entgegenhaltungen:
- WO-A-01/47919
- WO-A1-02/064575
- WO-A1-03/000256
- WO-A1-2004/101553
- WO-A1-2004/101556

## Beschreibung

Die vorliegende Erfindung betrifft ein Verfahren zur Herstellung von 4-(4-Aminophenyl)-3-morpholinon durch Umsetzung von 4-(4-Nitrophenyl)-3-morpholinon mit Wasserstoff in Gegenwart eines Hydrierungskatalysators, dadurch gekennzeichnet, dass die Umsetzung in einem aliphatischen Alkohol erfolgt.

4-(4-Aminophenyl)-3-morpholinon ist ein zentrales Vorprodukt bei der Synthese von 5-Chlor-*N-*({(5*S*)-2-oxo-3-[4-(3-oxo-4-morpholinyl)-phenyl]-1,3-oxazolidin-5-yl}-methyl)-2-thiophencarboxamid, einem Inhibitor des Blutgerinnungsfaktors Xa, der zur Prophylaxe und/oder Behandlung verschiedener thromboembolischer Erkrankungen eingesetzt werden kann (siehe hierzu WO-A 01/47919).

Die Synthese von 5-Chlor-*N*-({(5*S*')-2-oxo-3-[4-(3-oxo-4-morpholinyl)-phenyl]-1,3-oxazolidin-5-yl}methyl)-2-thiophencarboxamid (IV) erfolgt gemäß WO-A 01/47919 ausgehend von 4-(4-Aminophenyl)-3-morpholinon (I), 2-[(2*S*)-2-Oxiranylmethyl]-1*H*-isoindol-1,3(2*H*)-dion (II) und 5-Chlorthiophen-2-carbonylchlorid (III]:

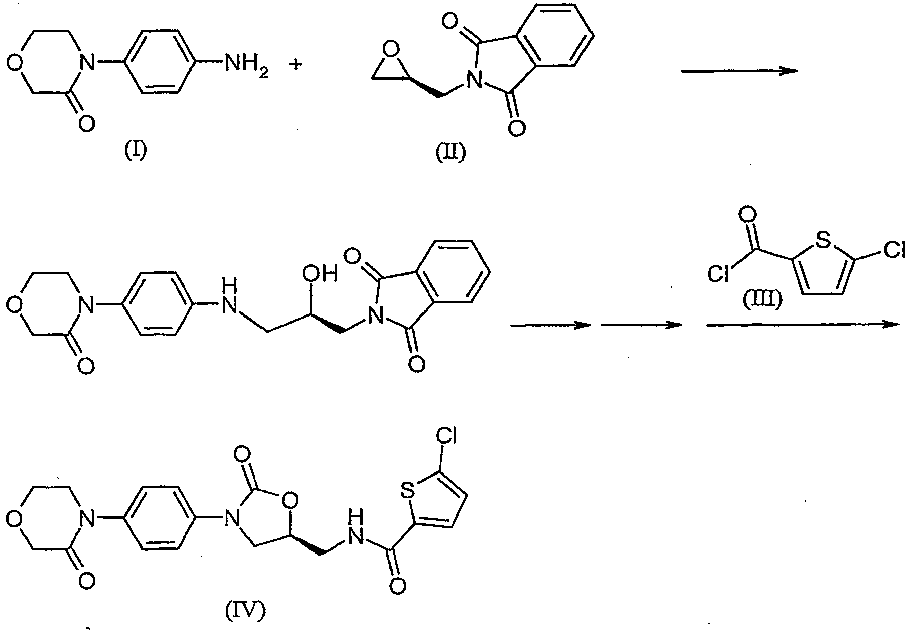

In WO-A 02/48099 ist 4-(4-Aminophenyl)-3-morpholinon ebenfalls als Vorstufe für die Synthese von Wirkstoffen beschrieben, es findet sich dort aber keinerlei Hinweis auf die Herstellung dieser Verbindung.

Hingegen ist in WO-A 01/47919 auch eine Herstellungsmethode für 4-(4-Aminophenyl)-3-morpholinon (I) beschrieben. Dabei wird zunächst Morpholin-3-on (V) mit Natriumhydrid deprotoniert und dann mit 4-Fluornitrobenzol (VI) zu 4-(4-Nitrophenyl)-3-morpholinon (VII) umgesetzt. Durch katalytische Hydrierung von (VII) mit Wasserstoff an Palladium auf Aktivkohle in Tetrahydrofuran als Lösungsmittel wird 4-(4-Aminophenyl)-3-morpholinon (I) erhalten:

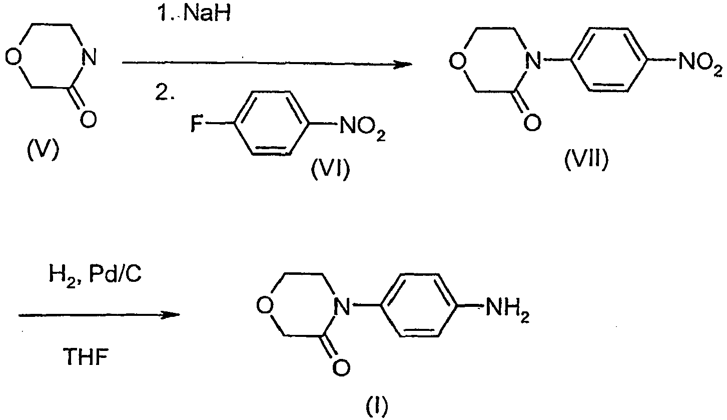

Allerdings.ist die Ausbeute dieses Verfahrens mit 17,6 % d. Th. in der ersten und 37,6 % d. Th. in der zweiten Stufe unbefriedigend. Bei der zweiten Stufe, der Hydrierung der Nitrogruppe von (VII), ist diese niedrige Ausbeute sicherlich auch durch die drastischen Reaktionsbedingungen, nämlich acht Stunden Reaktionszeit bei 70°C und einem Wasserstoffdruck von 50 bar, begründet. Der hohe Druck erfordert überdies erheblichen apparativen Aufwand. Das entstandene Produkt muss außerdem noch durch Kristallisation gereinigt werden. Diese Nachteile erschweren insbesondere die Umsetzung in größerem Maßstab.

Daraus ergibt sich die Aufgabe der vorliegenden Erfindung, ein vereinfachtes Verfahren zur Herstellung von 4-(4-Aminophenyl)-3-morpholinon (I) bereitzustellen, das insbesondere für die Herstellung größerer Mengen geeignet ist.

Überraschend wurde nun gefunden, dass man die Umsetzung von 4-(4-Nitrophenyl)-3-morpholinon (VII) mit Wasserstoff in Gegenwart eines Hydrierkatalysators, vorzugsweise Palladium auf Aktivkohle (5 %-ig), in aliphatischen Alkoholen, vorzugsweise in Alkoholen mit 1 bis 4 C-Atomen wie Methanol, Ethanol oder n-Butanol, durchführen kann. Besonders bevorzugt findet die Umsetzung in Ethanol in Lösung oder in Suspension statt. Durch die Verwendung von Ethanol als Lösungsmittel bei Temperaturen zwischen 40 und 120°C, vorzugsweise 75 bis 85°C, und einem Wasserstoffdruck von 2 bis 10 bar, vorzugsweise 4,5 bis 5,5 bar, kann die Reaktionszeit deutlich gesenkt werden. In der Regel ist die Umsetzung bereits nach etwa einer Stunde beendet. Diese milden Reaktionsbedingungen führen dazu, dass das Produkt (I) in hervorragender Ausbeute und in hoher Reinheit erhalten wird.

Im Fall von Ethanol als Lösungsmittel wird das Reaktionsgemisch zur Aufarbeitung lediglich mit Wasser und Ethanol versetzt und der Katalysator bei 40°C von der Produktlösung abfiltriert. Durch Einengen des Filtrats im Vakuum wird 4-(4-Aminophenyl)-3-morpholinon (I) isoliert. Bei der Verwendung anderer Lösungsmittel werden die Aufarbeitungsbedingungen entsprechend angepasst.

In einer bevorzugten Ausführungsform wird das produkthaltige Filtrat direkt weiter umgesetzt, ohne dass 4-(4-Aminophenyl)-3-morpholinon (I) in Substanz isoliert wird.

4-(4-Nitrophenyl)-3-morpholinon (VII) wird im Rahmen der vorliegenden Erfindung, anders als in WO-A 01/47919 beschrieben, durch Nitrierung von 4-Phenyl-3-morpholinon (VIII) hergestellt.

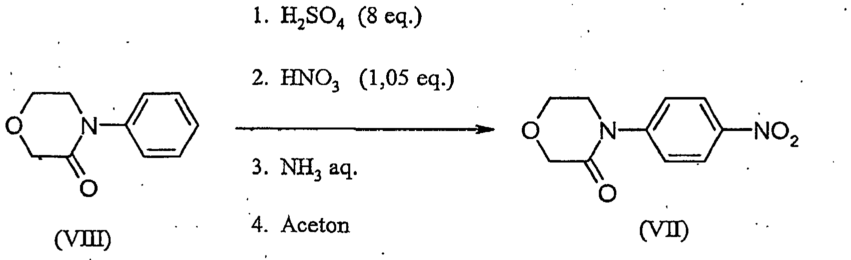

Hierbei wird 4-Phenyl-3-morpholinon (VIII) bei 5 bis 15°C Innentemperatur portionsweise zu 7 bis 8 Äquivalenten konzentrierter Schwefelsäure zugegeben und anschließend ca. 30 Minuten bei 25°C nachgerührt. Anschließend wird die Reaktionsmischung bei -10 bis 0°C mit 0,9 bis 1,2 Äquivalenten 65 %-iger Salpetersäure versetzt. Hierbei werden, wie bei Nitrierungen häufig, neben dem gewünschten para-Isomer auch die unerwünschten ortho- und meta-Isomeren gebildet. Zur Aufarbeitung werden bei 5 bis 15°C Wasser und 25 %-ige wässrige Ammoniaklösung zum Reaktionsgemisch zugegeben, bis ein pH-Wert von 7 bis 7,5 erreicht ist.

Überraschenderweise wurde nun gefunden, dass sich das gewünschte para-Isomer (VII) nach Zugabe von Aceton und Erwärmen des Reaktionsgemisches auf 40°C selektiv in der organischen Phase löst und auf diese Weise einfach und vorteilhaft durch Extraktion abgetrennt werden kann.

Beim Einengen der organischen Phase kristallisiert das Produkt (VII) aus dem Aceton/WasserGemisch aus und kann so isoliert werden.

Für die Herstellung von 4-Phenyl-3-morpholinon (VIII) sind in der Literatur verschiedene Synthesen beschrieben:

Gemäß US 3,092,630 werden 1,4-Dioxan-2-on und Anilin in einem Autoklaven bei 340°C umgesetzt, wobei eine gewisse, nicht näher ausgeführte Menge an (VIII) erhalten wird.

In J. Heterocycl. Chem. 2000, 37, 109 - 110 wird die Herstellung von (VIII) durch Phasentransferkatalysierte Oxidation von 4-Phenylmorpholin mit Kaliumpermanganat beschrieben. Als weiteres Reaktionsprodukt entsteht hier aber leichtentzündlicher Braunstein. Darüber hinaus beträgt die Ausbeute lediglich 45 % d. Th. und die Reaktion ist nur schwer in größerem Maßstab durchzuführen.

Die Umsetzung von 2-Chloressigsäureethylester mit 2-Anilinoethanol ist in Bull. Soc. Chim. France 1956, 1210 - 1212 sowie in Zhurnal Organicheskoi Khimii 1970, 6, 1305 - 1308 [CA 73:66523] beschrieben. Allerdings erfolgt hier die Deprotonierung mit Natrium in Toluol bzw. Benzol.

Die im Stand der Technik beschriebenen drastischen Reaktionsbedingungen oder technisch aufwendig handzuhabenden Reaktionsbedingungen, Reagenzien oder Lösungsmittel können vermieden werden, indem erfindungsgemäß 4-Phenyl-3-morpholinon (VIII) durch Umsetzung von Chloracetylchlorid mit 2-Anilinoethanol hergestellt wird.

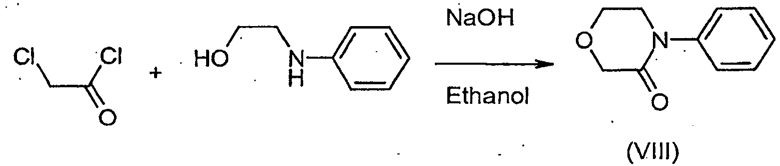

Dieses Verfahren ist insbesondere auch in technischem Maßstab gut anwendbar. Hierbei wird 2-Anilinoethanol in wässriger alkoholischer, vorzugsweise ethanolischer Lösung vorgelegt. 2,5 bis 3,5 Äquivalente Chloracetylchlorid und 4 bis 8, vorzugsweise 5 bis 7 Äquivalente Base werden gleichzeitig zudosiert. Als Base werden wässrige Alkali- oder Erdalkalihydroxidlösungen, vorzugsweise Natriumhydroxid- oder Kaliumhydroxidlösungen, insbesondere wässrige Natronlauge verwendet. Die Zugabe erfolgt bei einer Innentemperatur der Reaktionslösung von 30 bis 50°C, vorzugsweise von 35 bis 45°C. Außerdem wird die Geschwindigkeit der Zugabe so eingestellt, dass der pH-Wert der Reaktionslösung zwischen 10 und 13,5, vorzugsweise zwischen 12 und 12,5 liegt.

Nach Abkühlen der Reaktionslösung auf 0 bis 10°C kristallisiert das Produkt (VIII) aus und kann durch Filtration und Waschen mit kaltem Wasser in guter Ausbeute und hoher Reinheit erhalten werden.

Die Erfindung wird nachstehend durch ein bevorzugtes Ausführungsbeispiel näher erläutert. Soweit nicht anders angegeben, beziehen sich nachstehend alle Mengenangaben auf Gewichtsprozente.

### Synthese von 4-(4-Aminophenyl)-3-morpholinon (I)

### 1. Schritt: 4-Phenyl-3-morpholinon (VIII)

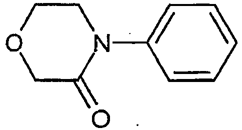

In einem 26 Liter-Kessel werden bei Raumtemperatur 1,65 kg (12,0 mol) 2-Anilinoethanol in 1,53 1 Ethanol gelöst und anschließend unter Rühren mit 4,58 1 Wasser versetzt. Die Lösung wird auf 38°C erwärmt. Dann werden gleichzeitig 4,07 kg (3,0 Äquivalente) Chloracetylchlorid und 6,60 kg 45 %-ige Natronlauge (6,2 Äquivalente) innerhalb von 60 bis 80 Minuten bei einer Innentemperatur von 38 bis 43°C zugegeben, so dass der pH-Wert zwischen 12 und 12,5 gehalten wird. Es wird 10 Minuten bei einem pH-Wert von 12 bis 12,5 nachgerührt, dann auf 2°C abgekühlt und 30 Minuten bei dieser Temperatur nachgerührt. Das ausgefallene Produkt wird abfiltriert und zweimal mit je 3,3 kg 2°C kaltem entsalzten Wasser nachgewaschen. Das Feuchtprodükt wird bei 50°C im Vakuum bis zur Massekonstanz getrocknet.
Ausbeute: 1700 g (80 % d. Th.) eines weißen Feststoffs.
Schmelzpunkt: 114°C.

### 2. Schritt: 4-(4-Nitrophenyl)-3-morpholinon (VII)

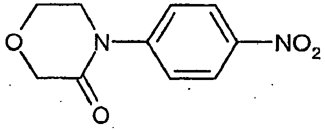

In einem 2 Liter-Kolben werden 177 g (1,0 mol) 4-Phenyl-3-morpholinon (VIII) bei 10°C Innentemperatur in vier Portionen in 728 g (7,4 Äquivalente) konzentrierte Schwefelsäure eingetragen. Danach wird auf 25°C erwärmt und 30 Minuten bei dieser Temperatur nachgerührt. Die Lösung wird auf -5°C abgekühlt und innerhalb einer Stunde mit 101,8 g (1,05 Äquivalente) 65 %-iger Salpetersäure versetzt. Es wird eine Stunde bei -5°C nachgerührt. In diese Lösung werden bei 10°C 1300 ml entsalztes Wasser zudosiert. Anschließend wird ebenfalls bei 10°C mit 25 %-iger wässriger Ammoniaklösung ein pH-Wert von 7,4 eingestellt. Die Suspension wird mit 2000 g Aceton versetzt und auf 40°C erwärmt. Dabei geht das Produkt in Lösung, so dass die Phasen getrennt werden können. Von der organischen Phase werden bei Normaldruck 1500 g Aceton/Wasser-Gemisch abdestilliert, wobei das Produkt ausfällt. Die Suspension wird auf 10°C abgekühlt, 30 Minuten nachgerührt und das Produkt isoliert. Das Feuchtprodukt wird mit 320 g kaltem Aceton gewaschen und im Vakuum bei 50°C getrocknet.
Ausbeute: 157 g (70 % d. Th.) eines weißen Feststoffs.
Schmelzpunkt: 152°C.

### 3. Schritt: 4-(4-Aminophenyl)-3-morpholinon (I)

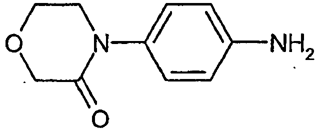

60 g (0,27 mol) 4-(4-Nitrophenyl)-3-morpholinon (VII) werden in 480 g Ethanol suspendiert, mit 3 g Palladium auf Aktivkohle (5 %-ig) versetzt und bei 80°C für eine Stunde mit 5 bar Wasserstoff beaufschlagt. Nach beendigter Hydrierung wird die Suspension mit 80 g Ethanol und 270 g Wasser versetzt, auf 40°C erwärmt und der Katalysator abfiltriert. Die Lösung wird im Vakuum eingeengt und der zurückbleibende Feststoff im Vakuum bei 50°C bis zur Gewichtskonstanz getrocknet.
Ausbeute: 48,4 g (93 % d. Th.) eines weißen bis leicht rötlich gefärbten Feststoffs.
Schmelzpunkt: 171 °C.

## Patentansprüche

1. Verfahren zur Herstellung von 4-(4-Aminophenyl)-3-morpholinon durch Umsetzung von 4-(4-Nitrophenyl)-3-morpholinon mit Wasserstoff in Gegenwart eines Hydrierungskatalysators, **dadurch gekennzeichnet, dass** die Umsetzung in einem aliphatischen Alkohol erfolgt.

2. Verfahren zur Herstellung von 4-(4-Aminophenyl)-3-morpholinon gemäß Anspruch 1, **dadurch gekennzeichnet, dass** der aliphatische Alkohol Ethanol ist.

3. Verfahren zur Herstellung von 4-(4-Aminophenyl)-3-morpholinon gemäß Anspruch 1 oder 2, **dadurch gekennzeichnet, dass** das produkthaltige Filtrat direkt weiter umgesetzt wird.

4. Verfahren zur Herstellung von 4-(4-Aminophenyl)-3-morpholinon gemäß einem der Ansprüche 1 bis 3, **dadurch gekennzeichnet, dass** 4-(4-Nitrophenyl)-3-morpholinon durch Nitrierung von 4-Phenyl-3-morpholinon hergestellt wird.

5. Verfahren gemäß Anspruch 4, **dadurch gekennzeichnet, dass** die Aufarbeitung des durch Nitrierung hergestellten 4-(4-Nitrophenyl)-3-morpholinons durch Extraktion mit Aceton erfolgt.

6. Verfahren gemäß Anspruch 5, **dadurch gekennzeichnet, dass** das 4-(4-Nitrophenyl)-3-morpholinon nach der Extraktion durch Kristallisation aus einem Aceton/Wasser-Gemisch isoliert wird.

7. Verfahren gemäß einem der Ansprüche 4 bis 6, **dadurch gekennzeichnet, dass** 4-Phenyl-3-morpholinon durch Umsetzung von 2-Anilinoethanol mit Chloracetylchlorid hergestellt wird.

8. Verfahren gemäß Anspruch 7, **dadurch gekennzeichnet, dass** Chloracetylchlorid und Base gleichzeitig zudosiert werden.

9. Verfahren gemäß Anspruch 8, **dadurch gekennzeichnet, dass** die Base wässrige Natronlauge ist.

10. Verfahren gemäß Anspruch 9, **dadurch gekennzeichnet, dass** die Zugabe von Chloracetylchlorid und Natronlauge bei einer Innentemperatur von 35 bis 45°C und unter Aufrechterhaltung eines pH-Werts der Reaktionslösung zwischen 12 und 12,5 erfolgt.

## Claims

1. Process for preparing 4-(4-aminophenyl)-3-morpholinone by reacting 4-(4-nitrophenyl)-3-morpholinone with hydrogen in the presence of a hydrogenation catalyst, **characterized in that** the reaction is effected in an aliphatic alcohol.

2. Process for preparing 4-(4-aminophenyl)-3-morpholinone according to Claim 1, **characterized in that** the aliphatic alcohol is ethanol.

3. Process for preparing 4-(4-aminophenyl)-3-morpholinone according to Claim 1 or 2, **characterized in that** the product-containing filtrate is reacted further directly.

4. Process for preparing 4-(4-aminophenyl)-3-morpholinone according to one of Claims 1 to 3, **characterized in that** 4-(4-nitrophenyl)-3-morpholinone is prepared by nitrating 4-phenyl-3-morpholinone.

5. Process according to Claim 4, **characterized in that** the 4-(4-nitrophenyl)-3-morpholinone prepared by nitration is worked up by extraction with acetone.

6. Process according to Claim 5, **characterized in that** the 4-(4-nitrophenyl)-3-morpholinone is isolated by crystallization from an acetone/water mixture after the extraction.

7. Process according to one of Claims 4 to 6, **characterized in that** 4-phenyl-3-morpholinone is prepared by reacting 2-anilinoethanol with chloroacetyl chloride.

8. Process according to Claim 7, **characterized in that** chloroacetyl chloride and base are metered in simultaneously.

9. Process according to Claim 8, **characterized in that** the base is aqueous sodium hydroxide solution.

10. Process according to Claim 9, **characterized in that** chloroacetyl chloride and sodium hydroxide solution are added at an internal temperature of 35 to 45°C and while maintaining a pH of the reaction solution between 12 and 12.5.

## Revendications

1. Procédé pour la préparation de 4-(4-aminophényl)-3-morpholinone par transformation de 4-(4-nitrophényl)-3-morpholinone avec de l'hydrogène en présence d'un catalyseur d'hydrogénation, **caractérisé en ce que** la transformation est réalisée dans un alcool aliphatique.

2. Procédé pour la préparation de 4-(4-aminophényl)-3-morpholinone selon la revendication 1, **caractérisé en ce que** l'alcool aliphatique est l'éthanol.

3. Procédé pour la préparation de 4-(4-aminophényl)-3-morpholinone selon la revendication 1 ou 2, **caractérisé en ce que** le filtrat contenant le produit est transformé directement davantage.

4. Procédé pour la préparation de 4-(4-aminophényl)-3-morpholinone selon l'une quelconque des revendications 1 à 3, **caractérisé en ce qu'**on prépare de la 4-(4-nitrophényl)-3-morpholinone par nitration de 4-phényl-3-morpholinone.

5. Procédé selon la revendication 4, **caractérisé en ce que** le traitement de la 4-(4-nitrophényl)-3-morpholinone préparée par nitration est réalisé par extraction avec de l'acétone.

6. Procédé selon la revendication 5, **caractérisé en ce que** la 4-(4-nitrophényl)-3-morpholinone est isolée, après l'extraction, par cristallisation à partir d'un mélange acétone/eau.

7. Procédé selon l'une quelconque des revendications 4 à 6, **caractérisé en ce que** la 4-phényl-3-morpholinone est préparée par transformation de 2-anilinoéthanol avec du chlorure de chloroacétyle.

8. Procédé selon la revendication 7, **caractérisé en ce que** le chlorure de chloroacétyle et la base sont dosés en même temps.

9. Procédé selon la revendication 8, **caractérisé en ce que** la base est la lessive de soude caustique aqueuse.

10. Procédé selon la revendication 9, **caractérisé en ce que** l'addition de chlorure de chloroacétyle et de lessive de soude caustique est réalisée à une température interne de 35 à 45°C et en maintenant un pH de la solution réactionnelle entre 12 et 12,5.
